# EUROPEAN PATENT APPLICATION

(11) **EP 3 178 379 A1**
(43) Date of publication of application: **14.06.2017**
(21) Application number: 15306963.8
(22) Date of filing: 09.12.2015
(51) Int. Cl.: A61B 5/00, A61B 5/16, A61N 7/00

(54) **METHOD AND DEVICE FOR BIOELECTRIC PHYSIOLOGICAL SIGNAL ACQUISITION AND PROCESSING**

(71) Applicant: RYTHM, 75009 Paris (FR)
(72) Inventor: SOULET DE BRUGIÈRE, Quentin, 33115 Pyla sur Mer (FR); MERCIER, Hugo, 91380 Chilla Mazarin (FR); MOHAMADABADI, Kaveh, 75016 Paris (FR)
(74) Representative: Cabinet Plasseraud

(57) **Abstract**

A method for bioelectric physiological signal acquisition and processing comprising:
- a first signal processing operation comprising:
/a/ acquiring a first analog signal,
/b/ converting the first analog signal in a first digital signal with a first bit depth,
/c/ transferring the first digital signal with the first bit depth to a microprocessor;

- upon reception of a signal to switch from a first operating mode to a second operating mode, a second signal processing operation comprising:
/e/ acquiring a second analog signal,
/f/ converting the second analog signal in a second digital signal with a first bit depth,
/g/ converting the second digital signal with the first bit depth to a converted second digital signal with a second bit depth,
/h/ transferring the converted second digital signal to the microprocessor, and
/i/ processing the transferred signal on the microprocessor.

## Description

### FIELD OF THE INVENTION

The instant invention relates to methods and devices for bioelectric physiological signal acquisition and processing.

### BACKGROUND OF THE INVENTION

Bioelectric physiological signals generated by electrical currents within the neurons or the nerves of an individual can be detected by performing electrocardiogram or electroencephalogram for instance. Such measurements have led to breakthrough in the field of biological sciences and present huge potentials in many daily-life fields such as self-quantization, self-optimization or human-computer interfaces to name only a few.

Recently, bioelectric physiological signal acquisition devices have started to be developed with the aim of providing conformable and easy to use ways of acquiring and processing bioelectric physiological signal.

Documents WO 2015/017563, CN 103961093, CN 102512159 and WO 2014062738 describe examples of such devices.

These devices usually comprise a few electrodes to acquire an analog signal, an acquisition chain to convert the signal in a digital signal and a microprocessor to process the signals. These devices are designed to be worn by the user during a certain period, for example a period of sleep in the case of sleep tracking devices. These devices may also act on the brain function of the user during this period, for example by emitting sound pattern to stimulate the brain of the user during sleep.

Since these devices may be worn over long periods of time, up to several hours, there is an incentive for the devices to be autonomous and able to process signals without having to communicate with external servers. This way, it is possible to minimize exposure of the user to electromagnetic fields.

However, the computational power and memory required to process the bioelectric signals imposes stringent demands on the quantity of energy stored in the batteries and/or the autonomy of the devices.

There is thus a need to further improve the autonomy of these bioelectric physiological signal acquisition and processing devices while keeping a high quality and reliability of signals acquisition.

The instant invention has notably for object to improve this situation.

### SUMMARY OF THE INVENTION

To this aim, a first object of the invention is a method for bioelectric physiological signal acquisition and processing comprising:
- providing an autonomous bioelectric physiological signal acquisition device comprising at least one electrode, at least one acquisition chain, a microcontroller connected to said at least one acquisition chain and a microprocessor;
- performing at least once a first signal processing operation comprising at least the following steps:
   /a/ acquiring a first analog signal representative of a bioelectric physiological signal by means of said at least one electrode,
   /b/ converting said first analog signal in a first digital signal with a first bit depth by means of said at least one acquisition chain,
   /c/ transferring said first digital signal with said first bit depth to the microprocessor;
- upon reception of a signal to switch from a first operating mode to a second operating mode on the microcontroller, performing at least once a second signal processing operation comprising at least the following steps:
   /e/ acquiring a second analog signal representative of a bioelectric physiological signal by means of said at least one electrode,
   /f/ converting said second analog signal in a second digital signal with said first bit depth by means of said at least one acquisition chain,
   /g/ converting said second digital signal with said first bit depth to a converted second digital signal with a second bit depth by means of said microcontroller,
   /h/ transferring said converted second digital signal to the microprocessor, and
   /i/ / processing the transferred converted second digital signal on the microprocessor.

In some embodiments, one might also use one or more of the following features:
- the second bit depth is lower than the first bit depth, in particular the first bit depth is a 24 bit depth and the second bit depth is a 16 bit depth;
- upon reception of a signal to switch to a second mode on the microcontroller, the microcontroller command the analog digital converters of the acquisition chain to switch from a high resolution mode to a low resolution mode;

- during the step of converting said first analog signal in a first digital signal, analog digital converters of the acquisition chain convert the first analog signal in a first digital signal with a first data rate,
   during the step of converting said second analog signal in a second digital signal, said analog digital converters of the acquisition chain convert the second analog signal in a second digital signal with a second data rate,
   wherein the first data rate is lower than the second data rate,
   in particular wherein the first data rate is 250 SPS and the second data rate is 500 SPS;
- the steps of converting the second digital signal from the first bit depth to the second bit depth and the step of transferring said converted signal to the microprocessor are performed simultaneously, in particular the second digital signal is not stored in a memory prior to being transferred to the microprocessor;
- the step of transferring the first digital signal to the microprocessor comprises the sub-steps of:
- storing a portion of the first digital signal in a temporary memory connected to the microcontroller, and
- periodically transferring said stored portion of the digital signal to the microprocessor;
- the step of transferring the first digital signal to the microprocessor further comprises the sub-step of processing the transferred signal on the microprocessor to determine whether the first analog signal is indicative of a first predefined mental state of an individual;
- the microprocessor is periodically awake to process the transferred first digital signal and set back in a sleep state after said processing;
- the first signal processing operation is reiterated continuously until the microprocessor determines that the first analog signal is indicative of a first predefined mental state of an individual, and
   if the microprocessor determines that the first analog signal is indicative of said first predefined mental state, the microprocessor sends a signal to switch to a second operating mode to the microcontroller;
- the step of processing the transferred converted second digital signal on the microprocessor comprises a sub-step of determining whether the second analog signal is indicative of a second predefined mental state of an individual,
   the second signal processing operation is reiterated continuously until the microprocessor determines that the second analog signal is indicative of said second predefined mental state, and
   if the microprocessor determines that the second analog signal is indicative of said second predefined mental state of an individual, the microprocessor sends a signal to switch to a first operating mode to the microcontroller, the first signal processing operation is performed at least once;
- the first signal processing operation and the second signal processing operation are reiterated continuously during a period of acquisition of the device;
- the step of processing the transferred converted second digital signal on the microprocessor further comprises a sub-step of commanding an acoustic transducer of the device to generate acoustic stimulations to an individual.

Another object of the invention is an autonomous bioelectric physiological signal acquisition device comprising
- at least one electrode able to acquire an analog signal representative of a bioelectric physiological signal,
- at least one acquisition chain associated to said electrode and able to convert an analog signal in a digital signal,
- a microcontroller connected to the at least one acquisition chain and a microprocessor,
   the microcontroller being able to
   . transfer a digital signal to the microprocessor with a first bit depth,
   . receive a signal to switch from a first operating mode to a second operating mode
   . convert a digital signal from a first bit depth to a second bit depth, and
   . transfer a converted digital signal to the microprocessor,
   the microprocessor being able to process the transferred converted second digital signal.

In an embodiment of the invention, the device further comprises at least one acoustic transducer able to generate an acoustic stimulation to an individual following a command from the microprocessor.

With these features, among other advantages, the autonomy of the bioelectric physiological signals acquisition is improved and a high quality and reliability of signals acquisition is provided.

### BRIEF DESCRIPTION OF THE DRAWINGS

Other characteristics and advantages of the invention will readily appear from the following description of several of its embodiments, provided as non-limitative examples, and of the accompanying drawings.

On the drawings:
- Fig. 1 is a schematic view in perspective of an autonomous bioelectric physiological signal acquisition device according to an embodiment of the invention,
- Fig. 2 is a flowchart detailing a method for bioelectric physiological signal acquisition according to an embodiment of the invention,
- Fig. 3 is a detailed view of the control module of the device of figure 1, showing a schematic of printed circuit boards and their main components.

On the different Figures, the same reference signs designate like or similar elements.

### DETAILED DESCRIPTION

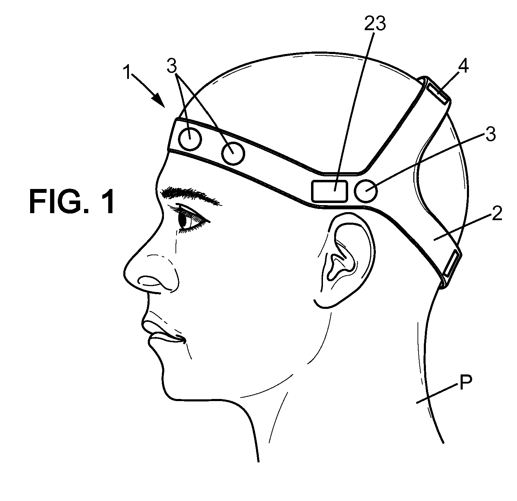

Figure 1 illustrates an autonomous bioelectric physiological signal acquisition device 1 according to an embodiment of the invention.

The device 1 is adapted to be worn by an individual P, for example during a sleep period of the individual.

The device 1 is for example adapted to be worn on the head of the individual P.

To this end, the device 1 can comprise one or more support members 2 adapted to at least partially surround the head of the individual so as to be held therein. In the embodiments of figure 1, the supports members 2 comprise three branches arranged so as to surround the head of the individual and maintain the device 1.

Alternatively, the device 1 may also be divided into elements, adapted to be worn on various parts of the body of the individual, for example on the head or wrist or on the chest.

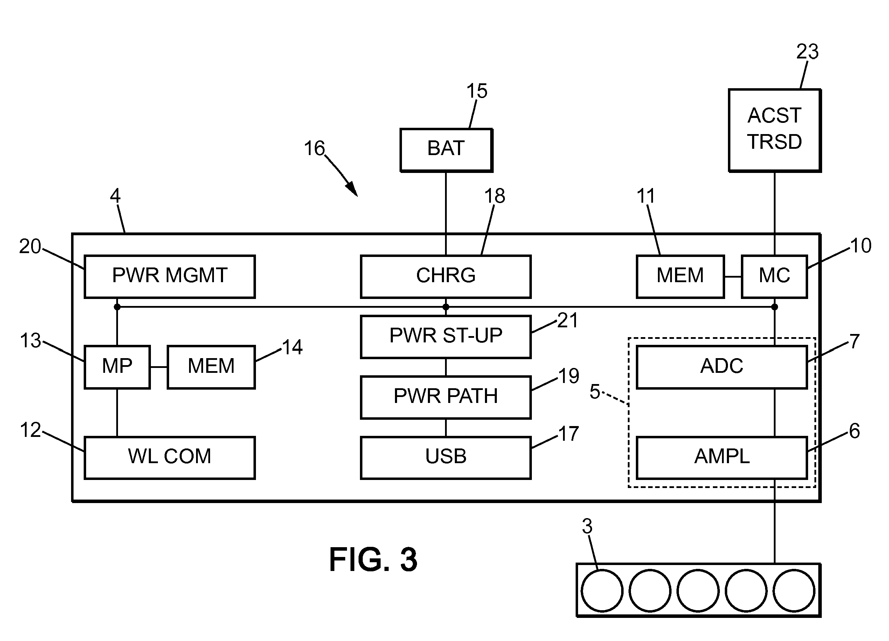

The device 1 of figure 1 and 3 comprises four measurement electrodes 3 able to be in contact with the skin of the individual and to acquire an analog signal. The analog signal can be representative of a bioelectric physiological signal.

Electrodes 3 may be reusable electrodes or disposable electrodes. Advantageously, electrodes 3 are reusable electrodes so as to simplify the daily use of the device. Electrodes 3 may be, in particular, dry electrodes. The electrodes 3 may also be of textile or silicone electrodes.

In one embodiment of the invention, the device 1 comprises five electrodes 3 arranged, for example on the surface of the scalp of the person P.

The five electrodes 3 may comprise for instance two reference electrodes located close to the mastoids, two measurement electrodes located on the forehead and a bias or virtual ground electrode that may also be located on the forehead. Such an arrangement is only described as a manner of example and should not be understood as being limitative with respect to the invention.

The five electrodes 3 are held by the support members 2.

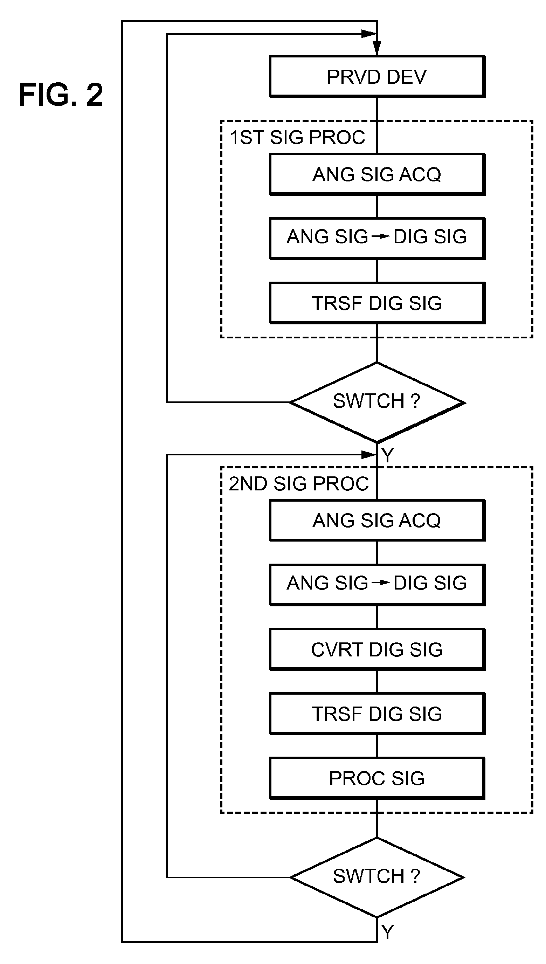

As illustrated on figures 2 and 3, the device 1 further comprises a control module 4 to process and store the signals of the electrodes.

The control module 4 comprises several acquisition chains 5, each acquisition chain 5 being associated to at least one electrode 3 of the plurality of electrodes, preferable two electrodes 3 of the plurality of electrodes to perform a differential measurement of the signal.

The device may for instance comprise four acquisition chains 5, each acquisition chain receiving signals from two electrodes 3, among the plurality of electrodes 3.

The acquisition chains 5 may be integrated in a single monolithic chip 5.

Each acquisition chain 5 comprises at least an analog digital converter 7. Preferably, each acquisition chain 5 also comprises a programmable differential analog amplifier 6.

The programmable differential analog amplifier 6 is able to amplify an analog signal acquired by one or two electrodes by an amplification factor.

The acquisition chains 5 may further comprise analog signal filters to filter the analog signal before said signal is amplified by the programmable analog amplifier 6. Such analog signal filters may comprise, for instance, low-pass and/or high-pass filters.

The input referred noise of the analog amplifiers 6 may be around 4uV at 150 Hz bandwidth. The programmable analog amplifiers 6 may thus have a high input impedance, high common mode rejection ratio, low bias current and low input referred noise to reduce noise sources such as flicker, low frequency noise, in particular noise from anti-aliasing, 50/60Hz power line, etc.

The analog amplifiers 6 may be programmable gain amplifiers with an adjustable gain, for instance adjustable to at least a 12 times factor of amplification.

The analog digital converters 7 are then able to convert the analog signal, in particular the amplified analog signal into a digital signal.

The analog digital converters 7 may comprise at least 4 channels. Each analog digital converter 7 may comprise a 24bit delta-sigma (Δ-Σ) type ADC with an output data rate between 250 SPS and 32 kSPS. A data rate is expressed as a number of samples per second (SPS) of a digital signal, for instance 250 samples per second.

The output data rate of the analog digital converters 7 corresponds to the data rate of the digital signal outputted by the analog digital converter 7.

The analog digital converters 7 may in particular be commanded to switch between a high resolution mode and a low resolution mode. The oversampling performed by the analog digital converters 7 may thus be adjustable. The switch between the high resolution mode and the low resolution mode may in particular control the conversion resolution and bandwidth of the digital signal.

In particular, the analog digital converters 7 may have an adjustable output data rate. A first output data rate, associated with the high resolution mode, may be lower than a second output data rate, associated with the low resolution mode.

The analog digital converters 7 may thus be able to selectively output a digital signal with a first data rate or a digital signal with a second data rate, the first data rate being lower than the second data rate.

The first output data rate may for instance correspond to a data rate of 250 SPS and the second output data rate may for instance correspond to a data rate of 500 SPS.

The accuracy of the analog to digital conversion performed by the analog digital converters 7 may be higher in the high resolution mode than in the low resolution mode.

The bandwidth of the digital signal may be smaller in the high resolution mode than in the low resolution mode.

With the first output data rate (high resolution mode), the power consumption of the analog digital converters 7, and thus of the acquisition chains 5, may also be higher than the power consumption with the second data rate (low resolution mode).

The four programmable analog amplifiers 6 and the four analog digital converters 7 of the four acquisition chains 5 may form a single monolithic chip 5.

One example of thus a monolithic chip 5 is an analog front-end module with reference ADS1294 of Texas Instrument®.

The control module 4 may further comprise a microcontroller 10 and a microprocessor 13.

The microcontroller 10 is able to continuously receive the digital signal from the acquisition chains 5.

The microcontroller 10 and the microprocessor 13 are able to communicate. In particular, the microcontroller 10 is able to transfer digital signal to the microprocessor 13. The microcontroller 10 is also able to receive from the microprocessor 13 a signal to switch between at least two operating modes as described further after, a first operating mode and a second operating mode.

The microprocessor 13 may be for instance a Cortex A9 Series Processor of ARM®. The microprocessor 13 may be able to achieve 2.5DMIPS/MHZ (with frequency at 1 GHz).

The microcontroller 10 may be a low power microcontroller. One example of low power microcontroller is a Cortex®-M0+ of ARM®.

The microcontroller 10 is further able to process a digital signal to change a bit depth of said digital signal.

A bit depth is a number of bits of information in each sample, for instance 24 bits of information per sample.

A digital signal with a high bit depth has a larger size than a digital signal with a lower bit depth. A digital signal with a high digital bit depth is thus more expensive to process, in term of computing power and computing time, than a digital signal with a lower bit depth.

A digital signal with a high bit depth also corresponds to a higher digital signal quality than a digital signal with a lower bit depth. Thus, a digital signal with a high bit depth is able to represent, and convey, higher frequency and lower amplitude signals than a digital signal with a lower bit depth.

The microcontroller 10 is able to convert a digital signal from a first bit depth to a second bit depth, the second bit depth being lower than the first bit depth.

A digital signal with the second bit depth will have a smaller size than the same digital signal with the first bit depth and will present a lower signal quality than the same digital signal with the first bit depth.

In one embodiment, the first bit depth may be 24 bits per sample and the second bit depth may be 16 bits per sample.

The first bit depth may be the native bit depth of the digital signal outputted by the acquisition chain 5. The second bit depth may be lower than the native bit depth of the digital signal outputted by the acquisition chain 5.

The microcontroller 10 may switch between a first operating mode and a second operating mode following the reception of a command from the microprocessor 13.

In the first operating mode, the microcontroller 10 may directly forward a digital signal with the first bit depth to the microprocessor 13.

In the second operating mode, the microcontroller 10 may convert the digital signal from the first bit depth to the second bit depth and transmit the converted signal to the microprocessor.

The microcontroller 10 may also be able to command the acquisition chain 5, in particular the analog digital converters 7, to switch between a high resolution mode and a low resolution mode as detailed above.

The microcontroller 10 may command the acquisition chain 5 to switch between said high resolution mode and low resolution mode following the reception of a command from the microprocessor 13.

In particular, the microcontroller 10 may command the acquisition chain 5 to operate in the high resolution mode when the microcontroller 10 operates in the first operating mode and to operate in the low resolution mode when the microcontroller 10 operates in the second operating mode.

The microcontroller 10 may additionally perform subsequent averaging of the digital data in order to obtain digital data with a lower output data rate, in particular when the acquisition chain 5 operate in the low resolution mode, for instance to convert a digital with a data rate of 250 SPS in a digital signal with a data rate of 125 SPS.

The microcontroller 10 may further be able to store the digital signal in a memory 11 of the device 1.

The memory 11 may be constituted of two modules: an internal memory located inside the chip of the microcontroller 10 and an external memory located outside the microcontroller 10, advantageously in proximity with the microcontroller 10. The external memory may have a larger capacity than the internal memory.

A method according to the invention may thus comprise a first signal processing operation comprising the following steps:
a step of acquiring a first analog signal representative of a bioelectric physiological signal by means of at least one electrode 3 of the device 1,
a step of converting said first analog signal in a first digital signal with a first bit depth by means of said at least one acquisition chain 5, and
a step of transferring said first digital signal to the microprocessor 13 with said first bit depth.

More precisely, the step of transferring the first digital signal to the microprocessor 13 may comprise the sub-steps of:
- storing a portion of the first digital signal in the memory 11 connected to the microcontroller 10, and
- periodically transferring said stored portion of the first digital signal to the microprocessor 13.

The step of transferring the first digital signal to the microprocessor 13 may further comprise a sub-step of processing the transferred signal on the microprocessor 13.

The microprocessor may then be able to determine whether the first analog signal is indicative of a predefined first mental state of an individual.

To this aim, the microprocessor 13 is able to perform pattern recognition of brain waves to infer mental states of an individual, for instance sleep phases. These mental states may then be compared with predefined mental states stored in a memory of the device.

In one embodiment of the invention, the device 1 may further comprise at least one acoustic transducer 23 able to generate an acoustic stimulation to stimulate the individual.

In this embodiment, a first predefined mental state may be a stimulation-ready state of the individual and a second predefined mental state may be a stimulation-averse state of the individual.

In the first predefined mental state it may be possible to acoustically stimulate the individual without any risk of waking up said individual. In the second predefined mental state, there might be a significantly higher risk of waking up the individual and an acoustic stimulation should thus not be performed.

The microprocessor 13 may be able to identify on the digital signal features that indicate that the individual is currently in a first predefined mental state or in a second predefined mental state.

In one embodiment, the microprocessor 13 may thus for instance be periodically awakened from a sleep state to receive the digital signal stored in the memory 11 and process said digital signal stored in the memory 11 and then send back in a sleep state after said processing.

The first signal processing operation may be reiterated continuously until the microprocessor 13 determines that the first analog signal is indicative of the first predefined mental state.

In this case, the microprocessor 13 may then send a signal to the microcontroller 10. This signal may indicate to the microcontroller 10 that it should switch from a first operating mode to a second operating mode.

As detailed above, the first operating mode corresponds to a first bit depth and the second operating mode corresponds to a second bit depth. In the first operating mode, the microcontroller 10 may transfer directly to the microprocessor 13 the raw data from the acquisition chain 5. In the second operating mode, the microcontroller 10 may convert the digital signal from the first bit depth to the second bit depth, for instance convert said digital signal from a 24 bit depth to a 16 bit depth. Moreover, the microcontroller 10 may also command the acquisition chain 5 to operate in the high resolution mode when the acquisition chain 5 operates in the first operating mode and to operate in the low resolution mode when the - acquisition chain 5 operates in the second operating mode.

Indeed, the amplitude and the frequency of the analog signals acquired by the electrodes is function of the mental state of the individual.

For instance, deep stages of sleep are usually associated with high amplitude and low frequency waves known as delta waves (0.1 - 4 Hz) while normal waking consciousness is associated with high frequency and low amplitude waves such as beta waves (12.5 - 30 Hz).

It is thus possible to process brain signals recorded during deep stages of sleep with a lower quality of signal than brain signals recorded during waking consciousness. The present invention take advantage of this feature to reduce the computing power and computing time required for operating during deep stage of sleep and thus extend the battery life time of the device.

In particular, the microprocessor 13 often consumes more power than the acquisition chain 5 and the microcontroller 10. Reducing the size of the digital data to process, and thus the time needed to process the data thus significantly lower the consumption of the device 1.

It is thus possible to dynamically adjust the quality of the signal to the mental state of the individual and the expected associated analog signal from the brain wave. It is also possible to dynamically adjust the consumption level to the quality of the electrode skin contact and to avoid saturated and non-usable signals while still having the highest possible signal levels.

Upon reception of the signal to switch to a second operating mode on the microcontroller, the method according to the invention may thus comprise a second signal processing operation comprising the following steps:
a step of acquiring a second analog signal representative of a bioelectric physiological signal by means of at least one electrode 3 of the device 1,
a step of converting said second analog signal in a second digital signal with a first bit depth by means of said at least one acquisition chain 5,
a step of converting said second digital signal in a converted second digital signal with a second bit depth by means of said microcontroller 10, and
a step of transferring said converted second digital signal to the microprocessor 13.

It should be noted that the steps of converting the second digital signal from the first bit depth to the second bit depth and transferring said converted signal to the microprocessor 13 may be performed simultaneously.

In particular, the second digital signal may not be stored in the memory 11 prior to being transferred to the microprocessor 13.

The microcontroller may thus be able to convert said second digital signal to the second bit depth on the fly while said digital signal is received from the acquisition chain 5 and send to the microprocessor 13.

Upon reception of the signal to switch to a second operating mode on the microcontroller, the method according to the invention may also comprise a step during which the microcontroller 10 command the acquisition chain 5 to switch from the high resolution mode to the low resolution mode.

Once the microprocessor 13 has received the converted second digital signal, it is then able to perform a step of processing said transferred converted second digital signal.

Said processing of the digital signal may be used to command the acoustic transducer 23 of the device 1 in function of the brain waves of the individual.

The step of processing the transferred converted second digital signal on the microprocessor 13 may thus comprise a sub-step of commanding said acoustic transducer 23 of the device to generate acoustic stimulations to the individual.

The step of processing the transferred converted second digital signal on the microprocessor 13 may also comprise a sub-step of determining whether the second analog signal is indicative of a second predefined mental state of an individual.

In particular, the second signal processing operation may be reiterated continuously until the microprocessor 13 determines that the second analog signal is indicative of said second predefined mental state.

In this case, the microprocessor 13 may then send a signal to the microcontroller 10. This signal may indicate to the microcontroller 10 that it should switch from the second operating mode to the first operating mode.

Upon reception of the signal to switch to the first operating mode on the microcontroller, the method according to the invention may also comprise a step during which the microcontroller 10 command the acquisition chain 5 to switch from the low resolution mode to the high resolution mode.

The method according to the invention may then goes back to performing the first signal processing operation which may again be reiterated continuously until the microprocessor 13 determines that the first analog signal is indicative of the first predefined mental state.

The first signal processing operation and the second signal processing operation may in particular be reiterated continuously during a period of acquisition of the device 1.

In one embodiment of the invention, the microprocessor 13 may store the signal in a second memory 14 of the device 1.

The second memory 14 may have a larger size than the temporary memory 11. The second memory 14 may comprise a double data rate (DDR) memory and an embedded multi-media controller (eMMC).

The device 1 may also comprise a wireless communication module 12. The microprocessor 13 may be able to control the wireless communication module 12. The wireless communication module 12 may comprises a RF transceiver chip 12 able to transmit and receive data using the Wi-Fi and the Bluetooth standards.

Moreover, the device 1 comprises at least one battery 15. The battery 15 powers the elements of the device 1, in particular at least the acquisition chains 5, the microcontroller 10 and the microprocessor 13, possibly through a power management chip 20.

The device may also comprise a power module 16 with a USB connector 17 and at least one battery charge chip 18. The battery charge chip 18 is able to receive energy from the USB connector 17 and charges said at least one battery 15.

In some embodiments, the device 1 may comprise two batteries 15 or more and the device 1 may then comprise two battery charge chips 18 respectively connected to each battery 15 and may comprise a power-path manager chip 19. The two batteries 15 may then be both connected to the power-path manager chip 19 through the respective battery charge chips 18.

## Claims

1. A method for bioelectric physiological signal acquisition and processing comprising:
- providing an autonomous bioelectric physiological signal acquisition device (1) comprising at least one electrode (3), at least one acquisition chain (5), a microcontroller (10) connected to said at least one acquisition chain (5) and a microprocessor (13);
- performing at least once a first signal processing operation comprising at least the following steps:
/a/ acquiring a first analog signal representative of a bioelectric physiological signal by means of said at least one electrode (3),
/b/ converting said first analog signal in a first digital signal with a first bit depth by means of said at least one acquisition chain (5),
/c/ transferring said first digital signal with said first bit depth to the microprocessor (13);
- upon reception of a signal to switch from a first operating mode to a second operating mode on the microcontroller (10), performing at least once a second signal processing operation comprising at least the following steps:
/e/ acquiring a second analog signal representative of a bioelectric physiological signal by means of said at least one electrode (3),
/f/ converting said second analog signal in a second digital signal with said first bit depth by means of said at least one acquisition chain (5),
/g/ converting said second digital signal with said first bit depth to a converted second digital signal with a second bit depth by means of said microcontroller (10),
/h/ transferring said converted second digital signal to the microprocessor (13), and
/i/ / processing the transferred converted second digital signal on the microprocessor (13).

2. The method of claim 1, wherein the second bit depth is lower than the first bit depth, in particular wherein the first bit depth is a 24 bit depth and the second bit depth is a 16 bit depth.

3. The method of claim 1 or 2, wherein upon reception of a signal to switch to a second mode on the microcontroller (10), the microcontroller (10) command the analog digital converters of the acquisition chain (5) to switch from a high resolution mode to a low resolution mode.

4. The method of anyone of claims 1 to 3,
wherein during the step of converting said first analog signal in a first digital signal, analog digital converters (7) of the acquisition chain (5) convert the first analog signal in a first digital signal with a first data rate,
wherein during the step of converting said second analog signal in a second digital signal, said analog digital converters (7) of the acquisition chain (5) convert the second analog signal in a second digital signal with a second data rate,
wherein the first data rate is lower than the second data rate,
in particular wherein the first data rate is 250 SPS and the second data rate is 500 SPS.

5. The method of anyone of claims 1 to 4, wherein the steps of converting the second digital signal from the first bit depth to the second bit depth and the step of transferring said converted signal to the microprocessor (13) are performed simultaneously, in particular wherein the second digital signal is not stored in a memory (11) prior to being transferred to the microprocessor (13).

6. The method of anyone of claims 1 to 5, wherein the step of transferring the first digital signal to the microprocessor (13) comprises the sub-steps of:
- storing a portion of the first digital signal in a temporary memory (11) connected to the microcontroller (13), and
- periodically transferring said stored portion of the digital signal to the microprocessor (13).

7. The method of anyone of claims 1 to 6, wherein the step of transferring the first digital signal to the microprocessor (13) further comprises the sub-step of processing the transferred signal on the microprocessor (13) to determine whether the first analog signal is indicative of a first predefined mental state of an individual.

8. The method of anyone of claims 1 to 7, wherein the microprocessor (13) is periodically awake to process the transferred first digital signal and set back in a sleep state after said processing.

9. The method of anyone of claims 1 to 8, wherein the first signal processing operation is reiterated continuously until the microprocessor (13) determines that the first analog signal is indicative of a first predefined mental state of an individual,
and wherein, if the microprocessor (13) determines that the first analog signal is indicative of said first predefined mental state, the microprocessor (13) sends a signal to switch to a second operating mode to the microcontroller (10).

10. The method of anyone of claims 1 to 9, wherein the step of processing the transferred converted second digital signal on the microprocessor (13) comprises a sub-step of determining whether the second analog signal is indicative of a second predefined mental state of an individual,
wherein the second signal processing operation is reiterated continuously until the microprocessor (13) determines that the second analog signal is indicative of said second predefined mental state,
and wherein, if the microprocessor (13) determines that the second analog signal is indicative of said second predefined mental state of an individual, the microprocessor (13) sends a signal to switch to a first operating mode to the microcontroller (10), the first signal processing operation is performed at least once.

11. The method of anyone of claims 1 to 10, wherein the first signal processing operation and the second signal processing operation are reiterated continuously during a period of acquisition of the device.

12. The method of anyone of claims 1 to 11, wherein the step of processing the transferred converted second digital signal on the microprocessor (13) further comprises a sub-step of commanding an acoustic transducer (23) of the device (1) to generate acoustic stimulations to an individual.

13. An autonomous bioelectric physiological signal acquisition and processing device comprising
- at least one electrode (3) able to acquire an analog signal representative of a bioelectric physiological signal,
- at least one acquisition chain (5) associated to said electrode (3) and able to convert an analog signal in a digital signal,
- a microcontroller (10) connected to the at least one acquisition chain (5) and a microprocessor (13),
the microcontroller (10) being able to
. transfer a digital signal to the microprocessor (13) with a first bit depth,
. receive a signal to switch from a first operating mode to a second operating mode
. convert a digital signal from a first bit depth to a second bit depth, and
. transfer a converted digital signal to the microprocessor,
the microprocessor (13) being able to process the transferred converted second digital signal.

14. Device according to claim 13, further comprising at least one acoustic transducer (23) able to generate an acoustic stimulation to an individual following a command from the microprocessor (13).
